(19) Europäisches Patentamt
European Patent Office
Office européen des brevets

(11) Veröffentlichungsnummer: **0 021 112**
**A1**

(12) # EUROPÄISCHE PATENTANMELDUNG

(21) Anmeldenummer: **80103020.6**

(22) Anmeldetag: **30.05.80**

(51) Int. Cl.[3]: **C 07 C 69/63**, C 07 C 55/40, C 07 C 51/60, C 07 C 67/14

(30) Priorität: **12.06.79 DE 2923775**

(43) Veröffentlichungstag der Anmeldung: **07.01.81 Patentblatt 81/1**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**
Erfinder: **Priesnitz, Uwe, Dr., Heinrich-Heine-Strasse 42, D-4750 Unna-Massen (DE)**
Erfinder: **Riebel, Hans-Jochem, Dr., In der Beek 92, D-5600 Wuppertal 1 (DE)**

(54) **Verfahren zur Herstellung von 1,3-Dibrom-2,2-dimethylpropan-1,3-dicarbonsäure-derivaten sowie 1,3-Dibrom-2,2-dimethyl-propan-1,3-dicarbonsäure-dichlorid- und -chlorid-ester als neue Zwischenprodukte.**

(57) Gegenstand der vorliegenden Erfindung sind ein neues Verfahren zur Herstellung von 1,3-Dibrom-2,2-dimethyl-propan-1,3-dicarbonsäure-derivaten, bei welchem man 2,2-Dimethyl-propan-1,3-dicarbonsäurederivate zunächst mit einem oder mehreren Säurehalogeniden, gegebenenfalls in Gegenwart eines Katalysators, und dann mit Brom bei Temperaturen zwischen 0 und 150° C umsetzt und gegebenenfalls die auf diese Weise gebildeten 1,3-Dibrom-2,2-dimethylpropan-1,3-dicarbonsäure-chloride und/oder -chlorid-ester durch Umsetzung mit Alkoholen nach üblichen Methoden verestert, das dadurch gekennzeichnet ist, daß man als Säurehalogenide Säure-chloride einsetzt, sowie neue 1,3-Dibrom-2,2-dimethyl-propan-1,3-dicarbonsäure-chloride und -chlorid-ester.

EP 0 021 112 A1

BAYER AKTIENGESELLSCHAFT
Zentralbereich
Patente, Marken und Lizenzen

5090 Leverkusen, Bayerwerk
Rt/Kü
Typ IVa/ZP

Verfahren zur Herstellung von 1,3-Dibrom-2,2-dimethyl-propan-1,3-dicarbonsäure-derivaten sowie 1,3-Dibrom-2,2-dimethyl-propan-1,3-dicarbonsäure-dichlorid- und -chlorid-ester als neue Zwischenprodukte

Die Erfindung betrifft ein neues Verfahren zur Herstellung von teilweise bekannten 1,3-Dibrom-2,2-dimethyl-propan-1,3-dicarbonsäure-derivaten, sowie 1,3-Dibrom-2,2-dimethyl-propan-1,3-dicarbonsäure-dichlorid und -chlorid-ester als neue Zwischenprodukte in diesem Verfahren.

Es ist bereits bekannt, daß man 1,3-Dibrom-2,2-dimethyl-propan-1,3-dicarbonsäure-diäthylester erhält, wenn man 2,2-Dimethyl-propan-1,3-dicarbonsäure-anhydrid (β,β-Dimethyl-glutarsäure-anhydrid) mit Phosphorpentabromid und Brom umsetzt und das Halogenierungsprodukt mit Äthanol zur Reaktion bringt (vergleiche J.Chem.Soc. (London) 79 (19o1), 754).



BAD ORIGINAL

Die Verwendung des teuren und wenig stabilen Phosphorpentabromids ist offenbar durch das Vorurteil bedingt, daß die Umsetzung mit Brom nur in Gegenwart eines Säurebromids selektiv zum gewünschten 1,3-Dibrom-2,2-dimethyl-propan-dicarbonsäure-derivat führen könne.

Wegen der möglichen Bedeutung von 1,3-Dibrom-2,2-dimethyl-propan-1,3-dicarbonsäure-derivaten als Zwischenprodukte für Pyrethroide bestand nun Interesse an einem Verfahren, nach welchem diese Verbindungen in guter Ausbeute und hoher Reinheit bei vertretbarem technischem Aufwand herzustellen sind.

Gegenstand der vorliegenden Erfindung sind

(1) ein neues Verfahren zur Herstellung von 1,3-Dibrom-2,2-dimethyl-propan-1,3-dicarbonsäurederivaten der Formel I

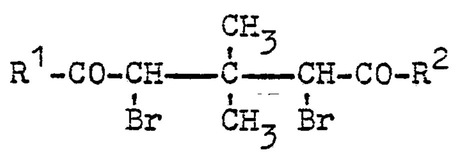

(I)

in welcher

$R^1$ und $R^2$ gleich oder verschieden sind und für Chlor oder Alkoxy stehen,

bei welchem man 2,2-Dimethyl-propan-1,3-dicarbonsäurederivate der Formel II

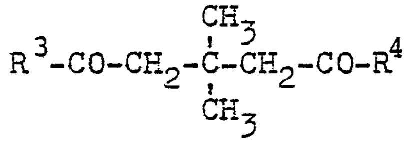

(II)

in welcher

$R^3$ für Hydroxy oder Alkoxy steht und

$R^4$ für Hydroxy oder zusammen mit $R^3$ für ein Anhydrid-Sauerstoffatom steht,

zunächst mit einem oder mehreren Säurehalogeniden, gegebenenfalls in Gegenwart eines Katalysators, und dann mit Brom bei Temperaturen zwischen 0 und 15o °C umsetzt und gegebenenfalls die auf diese Weise gebildeten 1,3-Dibrom-2,2-dimethyl-propan-1,3-dicarbonsäure-chloride und/oder -chlorid-ester der Formel I a

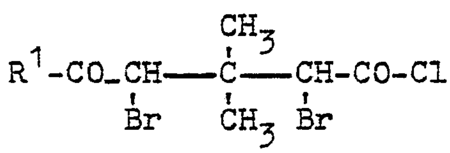

(I a)

in welcher

$R^1$ für Chlor oder Alkoxy steht,

durch Umsetzung mit Alkoholen nach üblichen Methoden verestert,
dadurch gekennzeichnet, daß man als Säurehalogenide Säure-c h l o r i d e einsetzt;

(2) neue 1,3-Dibrom-2,2-dimethyl-propan-1,3-dicarbonsäure-chloride und -chlorid-ester der Formel I a

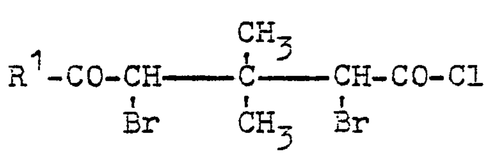

(I a)

in welcher

$R^1$ für Chlor oder Alkoxy steht.

Es ist als überraschend zu bezeichnen, daß nach dem neuen Verfahren 1,3-Dibrom-2,2-dimethyl-propan-1,3-dicarbonsäure-derivate der Formel (I) bzw. (I a) mit sehr guten Ausbeuten und in hoher Reinheit erhalten werden.

Ein wesentlicher Vorteil des neuen Verfahrens besteht darin, daß Säurechloride an Stelle von Phosphorpentabromid verwendet werden können.

Verwendet man als Ausgangsverbindung beispielsweise 3,3-Dimethyl-propan-1,3-dicarbonsäure und als Halogenierungsmittel Phosphorpentachlorid und Brom, sowie als Veresterungsmittel Methanol, so können die Reaktionen dieser Verbindungen gemäß dem erfindungsgemäßen Verfahren durch folgendes Formelschema skizziert werden:

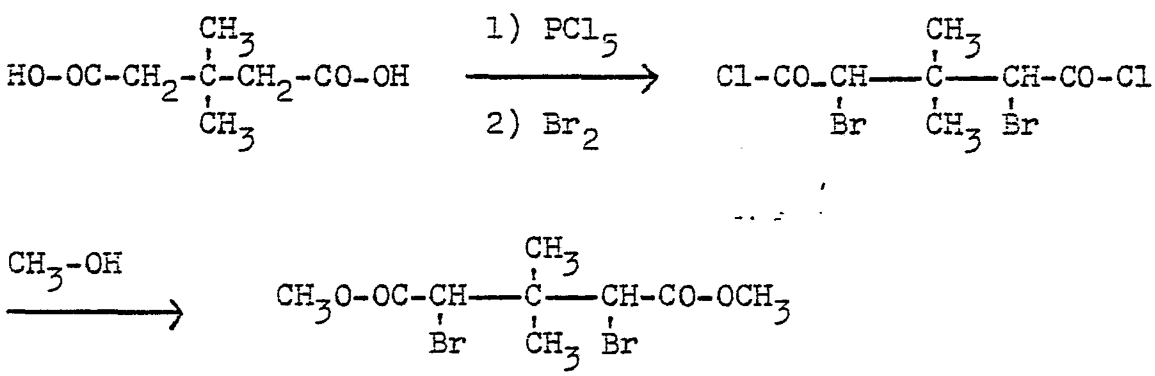

Die neuen 1,3-Dibrom-2,2-dimethyl-propan-1,3-dicarbonsäure-derivate sind durch Formel (I a) definiert. Vorzugsweise steht darin

$R^1$ für Chlor oder $C_1$-$C_4$-Alkoxy, insbesondere für Methoxy und Äthoxy.

Als Beispiele für die neuen Verbindungen der Formel (I a) seien genannt:
1,3-Dibrom-2,2-dimethyl-propan-1,3-dicarbonsäure-dichlorid, 1,3-Dibrom-2,2-dimethyl-propan-1,3-dicarbonsäure-chlorid-methylester und 1,3-Dibrom-2,2-dimethyl-propan-1,3-dicarbonsäure-chlorid-äthylester.

Die als Ausgangsstoffe einzusetzenden 2,2-Dimethyl-propan-1,3-dicarbonsäure-derivate sind durch Formel (II) definiert. Vorzugsweise stehen darin

$R^3$ für Hydroxy oder $C_1$-$C_4$-Alkoxy, insbesondere für Methoxy und

$R^4$ für Hydroxy oder zusammen mit $R^3$ für ein Anhydrid-Sauerstoffatom.

Als Beispiele für die Ausgangsverbindungen der Formel (II) seien genannt:
2,2-Dimethyl-propan-1,3-dicarbonsäure,
2,2-Dimethyl-propan-1,3-dicarbonsäure-anhydrid,
2,2-Dimethyl-propan-1,3-dicarbonsäure-monomethylester, -monoäthylester, -mono-n-propylester, -mono-iso-propylester, -mono-n-butylester, -mono-iso-butylester, -mono-sek.-butylester und -mono-tert.-butylester.

2,2-Dimethyl-propan-1,3-dicarbonsäure-derivate der Formel (II) sind bekannt (vergleiche J.Org.Chem. 15 (1950), 850 und 855).

Als Säurechloride, welche beim erfindungsgemäßen Verfahren eingesetzt werden können, seien beispielsweise Phosphorpentachlorid, Phosphoroxychlorid, Phosphortrichlorid, Thionylchlorid und Phosgen genannt. Vorzugsweise werden Phosphorpentachlorid und Thionylchlorid verwendet.

Das erfindungsgemäße Verfahren wird gegebenenfalls unter Verwendung von Katalysatoren durchgeführt. Als solche kommen Verbindungen in Betracht, welche gewöhnlich bei der Herstellung von Carbonsäurechloriden als Katalysatoren eingesetzt werden. Hierzu gehören insbesondere tertiäre und heterocyclische Amine wie z.B. Triäthylamin, N,N-Dimethyl-anilin und Pyridin sowie Ammoniumsalze wie z.B. Tetraäthylammoniumchlorid, Carbonsäureamide wie z.B.Dimethylformamid, Dimethylacetamid und N-Methyl-pyrrolidon, ferner Phosphorverbindungen wie z.B. Triphenylphosphin, Triphenylphosphinoxid oder Hexamethyl-phosphorsäuretriamid.

In einer bevorzugten Variante (a) des erfindungsgemäßen Verfahrens (1) wird 2,2-Dimethyl-propan-1,3-dicarbonsäureanhydrid (ß,ß-Dimethyl-glutarsäureanhydrid) mit etwa der äquimolaren Menge (0,9 bis 1,1 Moläquivalenten) Phosphorpentachlorid auf eine Temperatur zwischen 80 und 130 °C erhitzt und nach etwa 30 Minuten bei dieser Temperatur etwa die stöchiometrische Menge Brom (1,9 bis 2,2 Mol Brom je Mol Anhydrid) dazu gegeben.



BAD ORIGINAL

In einer zweiten bevorzugten Variante (b) von Verfahren (1) wird ein 2,2-Dimethyl-propan-1,3-dicarbonsäure-mono-ester (ß,ß-Dimethyl-glutarsäure-mono-ester) mit etwa 1,2 bis 3 Moläquivalenten Thionylchlorid, gegebenenfalls in Gegenwart eines der oben angegebenen Katalysatoren, auf eine Temperatur zwischen 4o und 9o°C erwärmt und nach etwa einer Stunde etwa die stöchiometrische Menge Brom (1,9 bis 2,2-Mol Brom je Mol Chlorid-ester) bei einer Temperatur zwischen 7o und 9o°C dazu gegeben.

In einer dritten bevorzugten Variante (c) von Verfahren (1) wird 2,2-Dimethyl-propan-1,3-dicarbonsäure (ß,ß-Dimethylglutarsäure) mit etwa 1,2 bis 3 Moläquivalenten Thionylchlorid, gegebenenfalls in Gegenwart eines der oben angegebenen Katalysatoren, auf eine Temperatur zwischen 4o und 9o°C erwärmt, nach etwa 3o Minuten bei dieser Temperatur mit 1 bis 1,3 Moläquivalenten Phosphorpentachlorid, anschließend bei einer Temperatur zwischen 8o und 13o°C mit etwa der stöchiometrischen Menge Brom (1,9 bis 2,2 Mol Brom je Mol Säure) versetzt und bei dieser Temperatur 15 bis 2o Stunden gerührt.

In einer vierten bevorzugten Variante (d) von Verfahren (1) wird 2,2-Dimethylpropan-1,3-dicarbonsäure (ß,ß-Dimethyl-glutarsäure) mit 1,9 bis 2,2 Moläquivalenten Phosphorpentachlorid bei Temperaturen zwischen 1o und 5o°C umgesetzt; das Reaktionsgemisch wird bei Temperaturen zwischen 8o und 13o°C mit etwa der stöchiometrischen Menge Brom (1,9 bis 2,2 Mol Brom je Mol Säure) versetzt und die Mischung wird bei dieser Temperatur 15 bis 2o Stunden gerührt.

Die nach den Verfahrensvarianten (a) bis (d) erhaltenen 1,3-Dibrom-2,2-dimethyl-propan-dicarbonsäurechloride bzw. -chlorid-ester der Formel (I a) können durch Destillation gereinigt werden. Die Säurechloride (I a) können jedoch auch ohne Zwischenisolierung, gegebenenfalls nach Abdestillieren überschüssiger Halogenierungsmittel, nach üblichen Methoden durch Umsetzung mit Alkoholen bei Temperaturen zwischen 1o und 8o°C verestert werden.

Zur Reinigung und Isolierung der Ester der Formel (I) wird mit Wasser verdünnt und mit einem mit Wasser nicht mischbaren Lösungsmittel, wie z.B. Äther oder Toluol, extrahiert. Die organischen Extrakte werden neutral gewaschen, getrocknet und eingeengt. Die im Rückstand verbleibenden Produkte können durch Vakuumdestillation gereinigt werden. Zur Charakterisierung dient der Siedepunkt.

Die nach dem erfindungsgemäßen Verfahren herzustellenden 1,3-Dibrom-2,2-dimethyl-propan-1,3-dicarbonsäureester der Formel (I) können zur Herstellung von 3,3-Dimethyl-cyclopropan-1,2-dicarbonsäureestern (Caronsäureestern) verwendet werden. Hierzu werden Ester der Formel (I) mit etwa 1,5-2 Moläquivalenten Zink, gegebenenfalls in Gegenwart eines Verdünnungsmittels wie z.B. Dimethylformamid, bei Temperaturen zwischen 2o und 2oo°C, vorzugsweise zwischen 8o und 15o°C, umgesetzt.

Zur Aufarbeitung wird mit einem mit Wasser nicht mischbaren Lösungsmittel, wie z.B. Toluol, verdünnt und abgesaugt. Das Filtrat wird mit verdünnter Salzsäure, dann mit Wasser gewaschen, getrocknet und eingeengt. Das Produkt kann durch Vakuumdestillation gereinigt werden.

3,3-Dimethyl-cyclopropan-1,2-dicarbonsäure-derivate können als Zwischenprodukte zur Herstellung von insektizid und akarizid wirksamen Pyrethroiden verwendet werden (vergleiche Pestic.Sci. 7 (1976), 492-498; Tetrahedron Lett. 1978, 1847-185o).

## Herstellungsbeispiele

**Beispiel 1:** 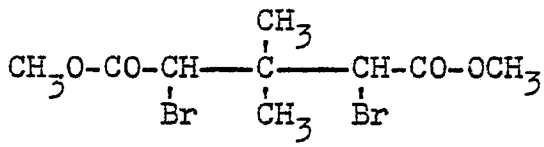

Eine Mischung von 28,4 g (o,2 Mol) ß,ß-Dimethyl-glutarsäureanhydrid und 41,6 g (o,2 Mol) Phosphorpentachlorid wird eine halbe Stunde auf 115-12o°C erhitzt. Dann werden bei dieser Temperatur 64 g (o,4 Mol) Brom zugetropft. Es tritt sofortige Entfärbung ein. Das Reaktionsgemisch wird auf 2o°C abgekühlt. Bei dieser Temperatur werden 3oo ml Methanol zugetropft. Nach 12-stündiger Nachreaktion bei 2o°C wird in 6oo ml Wasser gegossen. Es wird zweimal mit je 2oo ml Äther extrahiert. Die vereinigten Ätherextrakte werden mit 2oo ml 2%iger Natriumcarbonatlösung gewaschen, über Natriumsulfat getrocknet und eingedampft. Der Rückstand wird destilliert. Man erhält 55 g (79% der Theorie) 1,3-Dibrom-2,2-dimethyl-propan-1,3-dicarbonsäure-dimethylester in Form eines farblosen Öls vom Siedepunkt 1o5-1o8°C/1 mm Hg.

**Beispiel 2:**

1\. Stufe: 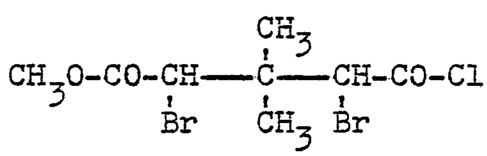

87 g (o,5 Mol) ß,ß-Dimethyl-glutarsäure-mono-methylester (Darstellung s. S.F.Birch et al., J. Chem. Soc. (London) 1952, 1364) werden zu 15o ml Thionylchlorid getropft und dann 1 Stunde unter Rückfluß gekocht.

Unter weiterem Sieden tropft man dann innerhalb von etwa 3 Stunden 17o g Brom zu und kocht nach Ende der Zugabe noch 1 Stunde nach. Das Reaktionsgemisch wird im Vakuum bei ca. 5o°C von überschüssigem Thionylchlorid befreit und dann im Vakuum destilliert. Man erhält auf diese Weise 145 g (83% der Theorie) 1,3-Dibrom-2,2-dimethyl-propan-1,3-dicarbonsäure-methylester-chlorid in Form eines farblosen Öles mit dem Siedepunkt 146-15o°C/ 11 mm Hg.

2. Stufe: 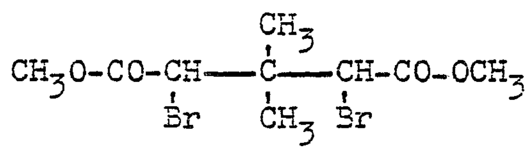

Ein Gemisch aus 62,5 g (ca. o,18 Mol) des oben beschriebenen Esterchlorides und 15o ml Methanol wird 2 Stunden bei Raumtemperatur gerührt, auf 5oo ml Wasser gegossen und 3 mal mit je 1oo ml Toluol extrahiert. Die organische Phase wird erst mit 5o ml gesättigter Natriumhydrogencarbonatlösung und dann mit 5o ml Wasser gewaschen, über Natriumsulfat getrocknet und im Vakuum eingedampft. Der Rückstand wird im Vakuum destilliert. Man erhält auf diese Weise 58 g (92% der Theorie) 1,3-Dibrom-2,2-dimethyl-propan-1,3-dicarbonsäuredimethylester in Form eines schwach rosa gefärbten Öles mit dem Siedepunkt 1o5-11o°C/ o,3 mm Hg.

<u>Beispiel 3:</u> 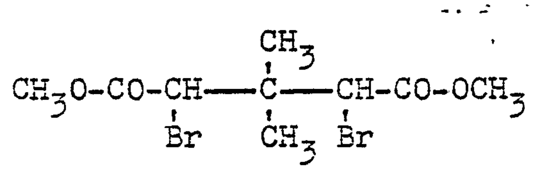

87 g (o,5 Mol) ß,ß-Dimethyl-glutarsäuremono-methylester (Darstellung s. S.F.Birch et al., J. Chem.Soc. (London)

1952, 1364) werden zu 15o ml Thionylchlorid getropft und dann 1 Stunde unter Rückfluß gekocht. Unter weiterem Sieden tropft man dann innerhalb von etwa 3 Stunden 17o g Brom zu und kocht nach Ende der Zugabe noch 1 Stunde nach. Das Reaktionsgemisch wird im Vakuum bei ca. 5o°C von überschüssigem Thionylchlorid befreit und dann zu 25o ml Methanol getropft, wobei die Temperatur bis zum Siedepunkt des Methanols ansteigt. Man rührt 1 Stunde nach und destilliert dann das Lösungsmittel im Vakuum ab. Der Rückstand wird in 4oo ml Toluol gelöst und 1 mal mit Natriumbicarbonatlösung und 2 mal mit Wasser gewaschen. Die organische Phase wird über Natriumsulfat getrocknet und im Vakuum eingeengt. Den Rückstand destilliert man im Hochvakuum. Man erhält so 138 g (3o% der Theorie) 1,3-Dibrom-2,2-dimethyl-propan-1,3-dicarbonsäure-dimethylester als farbloses Öl mit dem Siedepunkt 98-99°C/o,1 mm Hg.

Beispiel 4:

1. Stufe:

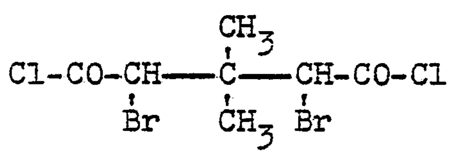

Ein Gemisch aus 9o ml Thionylchlorid und 48,1 g (o,3 Mol) ß,ß-Dimethyl-glutarsäure wird eine halbe Stunde unter Rückfluß gekocht, dann bei 5o-6o°C portionsweise mit 68,8 g (o,33 Mol) Phosphorpentachlorid und anschließend bei 9o-1oo°C mit 1oo,8 g (o,63 Mol) Brom versetzt. Man rührt 18 Stunden bei 1oo°C nach und fraktioniert das Reaktionsgemisch im Vakuum. Man erhält auf diese Weise 65 g (62% der Theorie) 1,3-Dibrom-2,2-dimethyl-propan-1,3-dicarbonsäuredichlorid mit dem Siedepunkt 128-132°C/ 7 mm Hg.

2. Stufe: 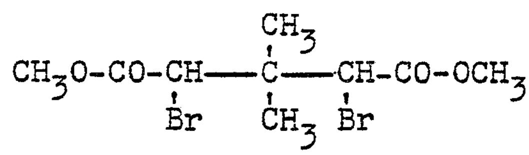

Eine Lösung von 17,8 g (o,o5 Mol) 1,3-Dibrom-2,2-dimethyl-propan-1,3-dicarbonsäure-dichlorid in 5o ml Methanol wird 2 Stunden gerührt und dann im Vakuum eingedampft. Den Rückstand löst man in loo ml Äther und schüttelt die Lösung erst mit 5o ml Wasser, dann mit 5o ml gesättigter Natriumhydrogencarbonatlösung und schließlich noch 2 mal mit je 5o ml Wasser. Nach Trocknen über Natriumsulfat wird das Lösungsmittel im Vakuum abgezogen und der Rückstand destilliert. Man erhält so 14,2 g (83% der Theorie) 1,3-Dibrom-2,2-dimethyl-propan-1,3-dicarbonsäure-dimethylester mit dem Siedepunkt loo-lo3°C/o,4 mm Hg.

Beispiel 5:

Zu 131 g (o,63 Mol) Phosphorpentachlorid gibt man portionsweise 48,1 g (o,3 Mol) Dimethyl-glutarsäure so zu, daß die Temperatur nicht über 5o°C steigt. Das so erhaltene Gemisch wird bei loo°C innerhalb von 3 Stunden mit loo,8 g (o,62 Mol) Brom versetzt und 2o Stunden bei loo°C nachgerührt. Nach Abdestillieren der flüchtigen Anteile tropft man das Produkt zu 15o ml Methanol und rührt 1 Stunde nach. Das Reaktionsgemisch wird mit 3oo ml Wasser versetzt und zwei mal mit je 25o ml Toluol ausgeschüttelt. Die organischen Phasen werden erst mit loo ml gesättigter Natriumhydrogencarbonatlösung und dann zwei mal mit je loo ml Wasser gewaschen, über Natriumsulfat getrocknet und im Vakuum eingedampft. Den Rückstand

destilliert man im Vakuum. Man erhält so 83 g (80% der Theorie) 1,3-Dibrom-2,2-dimethyl-propan-1,3-dicarbonsäure-dimethylester mit dem Siedepunkt 138-142°C/2 mm Hg.

Patentansprüche:

1. Verfahren zur Herstellung von 1,3-Dibrom-2,2-dimethyl-propan-1,3-dicarbonsäure-derivaten der Formel I

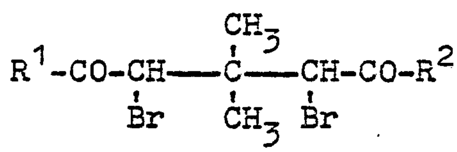 (I)

in welcher

$R^1$ und $R^2$ gleich oder verschieden sind und einzeln für Chlor oder Alkoxy stehen,

bei welchem man 2,2-Dimethyl-propan-1,3-dicarbonsäure-derivate der Formel II

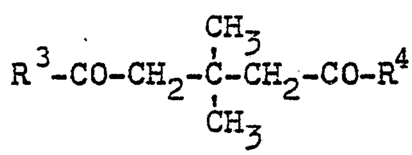 (II)

in welcher

$R^3$ für Hydroxy oder Alkoxy steht und

$R^4$ für Hydroxy oder zusammen mit $R^3$ für ein Anhydrid-Sauerstoffatom steht,

zunächst mit einem oder mehreren Säurehalogeniden, gegebenenfalls in Gegenwart eines Katalysators, und dann mit Brom bei Temperaturen zwischen 0 und 15o °C umsetzt und gegebenenfalls die auf diese Weise gebildeten 1,3-Dibrom-2,2-dimethyl-propan-1,3-dicarbonsäure-chloride und/oder -chlorid-ester der Formel I a

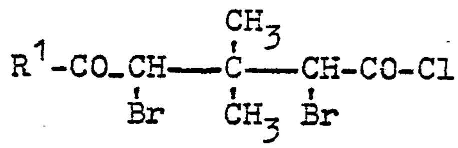

(I a)

in welcher

$R^1$ für Chlor oder Alkoxy steht,

durch Umsetzung mit Alkoholen nach üblichen Methoden verestert,
dadurch gekennzeichnet, daß man als Säurehalogenide Säure-c h l o r i d e einsetzt.

(2) 1,3-Dibrom-2,2-dimethyl-propan-1,3-dicarbonsäure-chloride und -chlorid-ester der Formel I a

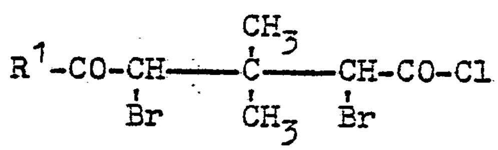

(I a)

in welcher
$R^1$ für Chlor oder Alkoxy steht.

Europäisches Patentamt

# EUROPÄISCHER RECHERCHENBERICHT

0021112

Nummer der Anmeldung

EP 80103020.6

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| D | JOURNAL OF THE CHEMICAL SOCIETY, 1901, Vol. LXXIX, Part II, (PERKIN, THORPE), Seiten 753-791<br>+ Seiten 754,755 +<br>---- | 1 | C 07 C 69/63<br>C 07 C 55/40<br>C 07 C 51/60<br>C 07 C 67/14 |

**RECHERCHIERTE SACHGEBIETE (Int. Cl. 3)**

C 07 C 69/00
C 07 C 55/00
C 07 C 51/00
C 07 C 67/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 31-07-1980 | HOFBAUER |